(19) Europäisches Patentamt
European Patent Office
Office européen des brevets

(11) EP 2 407 165 A1

(12) EUROPEAN PATENT APPLICATION
published in accordance with Art. 153(4) EPC

(43) Date of publication:
**18.01.2012 Bulletin 2012/03**

(21) Application number: **10750900.2**

(22) Date of filing: **11.03.2010**

(51) Int Cl.:
***A61K 31/4412*** *(2006.01)* ***A61K 8/49*** *(2006.01)*
***A61K 8/64*** *(2006.01)* ***A61K 36/00*** *(2006.01)*
***A61K 38/00*** *(2006.01)* ***A61P 9/00*** *(2006.01)*
***A61P 17/00*** *(2006.01)* ***A61P 17/18*** *(2006.01)*
***A61P 35/00*** *(2006.01)* ***A61P 39/06*** *(2006.01)*
***A61Q 19/02*** *(2006.01)*

(86) International application number:
**PCT/JP2010/054112**

(87) International publication number:
**WO 2010/104147 (16.09.2010 Gazette 2010/37)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.03.2009 JP 2009058636**

(71) Applicant: **HiPep Laboratories**
**Kyoto 602-8158 (JP)**

(72) Inventors:
- **NOKIHARA, Kiyoshi**
**Kyoto-shi**
**Kyoto 602-8158 (JP)**
- **MIYAZATO, Naeko**
**Kyoto-shi**
**Kyoto 602-8158 (JP)**

(74) Representative: **Wills, Andrew Jonathan et al**
**Mewburn Ellis LLP**
**33 Gutter Lane**
**London**
**EC2V 8AS (GB)**

# (54) ANTIOXIDANT AGENT

(57) Disclosed is an antioxidant which can exhibit a higher antioxidant ability than known antioxidative peptides. The antioxidant according to the present invention comprises mimosine and/or a mimosine-containing pcptide(s). The mimosine-containing peptide is a peptide which comprises at least one mimosine residue in its amino acid sequence. Mimosine has stronger antioxidant potency than known antioxidative tripeptides even when used as it is alone, and therefore is useful as an antioxidant. In addition, the antioxidant action of a peptide can be increased by introducing mimosine thereinto, thereby obtaining a peptide with higher antioxidant potency than the original one.

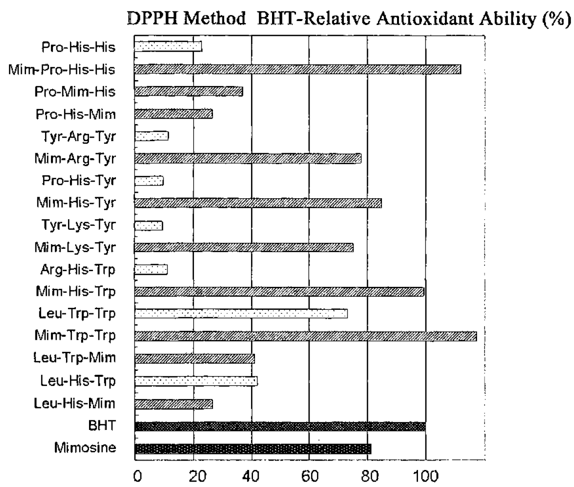


Fig.1

EP 2 407 165 A1

## Description

TECHNICAL FIELD

The present invention relates to an antioxidant comprising mimosine and/or a mimosine-containing peptide(s), and a tyrosinase inhibitor comprising a mimosine-containing peptide(s).

BACKGROUND ART

Human beings incorporate oxygen into their body at any time to preserve life. However, about 2-3% of the total amount of oxygen incorporated into the body becomes active oxygen. It is thought that excess active oxygen generated attacks lipids, carbohydrates, proteins and nucleic acids which consist of a living body and oxidizes them, by which tissues and cells are injured to result in cancer, stroke, aging, various lifestyle-relatcd diseases, skin aging (wrinkling), pigmentation (spotting) and the like. Therefore, removal of active oxygen in a living body is important for maintenance of health, and is thought to be a key to prevention of adult diseases. Hence, functional molecules having antioxidant ability began to be focused recently (Non-patent Document 1).

On the other hand, peptides which consist of natural amino acids and have an antioxidant action were discovered and analyzed for the structure more than 10 years ago. Based on their structure, researches were conducted using various synthetic peptides, which revealed that tripeptides play a central role in the activity (Non-patent Document 2).

Gin-Nemu (English name: Leucaena, scientific name: *Leucaena leucocephala* de Wit) is a legume plant flourishing in tropical and subtropical areas of the world, and contains a non-proteinogenic amino acid mimosine in large quantities. This subtropical plant Leucaena exerts an allelopathic effect to inhibit growth of other plants in its natural growth area, and it is known that mimosine generated in Leucaena is responsible for the allelopathic effect (Non-patent Document 3). Nokihara *et al.* have established the isolation and purification process, the quality control procedure and the derivatization technique for mimosine, and filed a patent application (Japanese Patent Application No. 2008-210592). Because of a lot of biological actions, mimosine is expected to be useful in various fields such as drug, agrochemical and functional cosmetic development (Patent Document 1, Non-patent Document 4).

Mimosine *per se* is a known compound. It is thought that high-value-added compounds can be created by introducing mimosine into a peptide to give various functions such as cell permeability, molecular recognition property and the like to mimosine. It is more advantageous to use mimosine in the form of such a high-value-added compound than use it as it is. For example, it is known that certain peptides can be used to give functions and/or effects to a cosmetic and that such a functionalization is sufficiently achieved at a peptide concentration of just several to 10 ppm in a cosmetic.

Based on peptides isolated from a soybean protein partial hydrolysate, the present inventors have completed a peptide (Pro-His-His) with high antioxidant potency. Furthermore, in the investigation of the analogous peptides, the inventors found that short peptides containing amino acids such as His, Trp and Tyr play an important role in the antioxidant activity. Focusing on the fact above, they have performed peptide designing, and elucidated the antioxidant action using synthetic peptides (Non-patent Documents 5 to 12). However, an antioxidant action of mimosine *per se* or mimosine-introduced peptides has not been reported yet.

PRIOR ART DOCUMENTS

PATENT DOCUMENTS

Patent Document 1: JP63-295502 A

NON-PATENT DOCUMENTS

Non-patent Document 1: Recent Development of Food Factors For The Disease Prevention, (eds. Niki, E., Yoshikawa, T., and Osawa, T.), CMC Publishers, Tokyo, 1998.

Non-patent Document 2: Chen, H-M., Muramoto, K., Yamauchi, F., and Nokihara, K., J. Agric. Food Chem., 44, 2619-2623, 1996. Antioxidant Activity of Designed Peptides Based on the Antioxidative Peptide Isolated from Digests of a Soybean Protein

Non-patent Document 3: Tawata S. Pesticide and Alternatives, Elservier Science Publishers, Amsterdam, pp. 541-544.

Non-patent Document 4: W.D. DeWys and T.C.Hall, europ. J. Cencer Vol.9, pp. 218-283. 1973.
Non-patent Document 5: Saito, K., Jin, D-H., Ogawa, T., Muramoto, K., Hatakeyama, E., Yasuhara. T. and Nokihara, K., J, Agric. Food Chemistry, 51, 3668-3674, 2003. Antioxidative Properties of Tripeptide Libraries Prepared by the Combinatorial Chemistry
Non-patent Document 6: Yasuhara, T., Nokihara, K., Furusho, T., and Kataoka, E., Jour. Agri. Sci., Tokyo Nogyo Daigaku, 43, 260-267, 1999. Construction of Synthetic Tripeptide Libraries and High-throughput Screening for Highly Antioxidative Peptides
Non-patent Document 7: Chen, H-M., Muramoto, K., Yamauchi, F., Fujimoto, K., and Nokihara, K., J. Agric. Food Chem., 46,49-53, 1998. Antioxidative Properties of Ilistidine-Containing Peptide Designed from Peptide Fragments Found in the Digests of a Soybean Protein
Non-patent Document 8: Chen, H M., Muramoto, K., Yamauchi, F., and Nokihara, K.,J. Agric. Food Chem., 44, 2619-2623, 1996. Antioxidant Activity of Designed Peptides Based on the Antioxidative Peptide Isolated from Digests of a Soybean Protein
Non-patent Document 9: Yasuhara, T., and Nokihara, K., Peptide 1997, ed. Shimonishi, Y., Kluwer Academic Publishers, The Netherlands, 134-135,1999. Manual Construction of Combinatorial Resin Bound Tripeptide Library (One Peptide on One Bead) and Screening to Find Antioxidative Peptides
Non-patent Document 10: Nokihara, K.. Yasuhara, T., Muramoto, K., Ando, E., and Wray, V., Peptide Chemistry 1996, ed. Kitada, C., Protein Research Foundation, Osaka, 245-248, 1997. Studies on Peptides Exhibiting Antioxidative Activity: Construction of a Peptide Library and Screening Non-patent Document 11:
Muramoto, K., and Nokihara, K., Recent Development of Food Factors For The Disease Prevention, (eds. Niki, E., Yoshikawa, T., and Osawa, T.) CMC Publishers, Tokyo, pp 159-166, 1998.
Non-patent Document 12: H-M. Chen et al., J. Agric. Food Chem (1995) 43, p.574-578

## SUMMARY OF THE INVENTION

### PROBLEMS TO BE SOLVED BYTHE INVENTION

Accordingly, an object of the present invention is to provide a peptide derivative which has a higher antioxidant ability than the known antioxidative peptides and is useful as an antioxidant.

### MEANS FOR SOLVING THE PROBLEMS

The present inventors have performed a number of investigations of functional peptides to find antioxidative peptides consisting of a small number of amino acids. The inventors have intensively studied based on these findings to find that a peptide with higher antioxidant potency can be obtained by introducing a mimosine residue(s) into a known antioxidative peptide, and that mimosine as it is also has antioxidant potency. Although it is known that mimosine as it is has a tyrosinase activity (Patent Document 1, Non-patent Document 4), the present inventors revealed for the first time that a peptide into which a mimosine residue(s) is(are) introduced has higher tyrosinase inhibitory activity than mimosine as it is, and can inhibit tyrosinase as strongly as kojic acid, a known potent tyrosinase inhibitor, and that therefore mimosine-introduced peptides are useful as a skin lightening agent. Thus, the present invention has been completed.

That is, the present invention provides an antioxidant comprising mimosine and/or a mimosine-containing peptide(s). The present invention also provides a method for improving an antioxidant action of a peptide, comprising substituting at least one amino acid in the peptide with mimosine, adding at least one mimosine to the peptide, and/or inserting at least one mimosine into the peptide. The present invention further provides a tyrosinase inhibitor comprising a mimosine-containing peptide(s). The present invention still further provides a composition for skin-lightening comprising as an effective ingredient the tyrosinase inhibitor according to the present invention. The present invention still further provides mimosine or a mimosine-containing peptide for use in antioxidation. The present invention still further provides a mimosine-containing peptide for use in tyrosinase inhibition.

The present invention still further provides a method for antioxidation of an article, comprising incorporating mimosine and/or a mimosine-containing peptidc(s) into an article in which antioxidation is desired. The present invention still further provides a method for removing active oxygen in a living body, comprising administering mimosine and/or a mimosine-containing peptide(s) to a living body. The present invention still further provides a method for inhibiting tyrosinase in a living body, comprising administering a mimosine-containing peptide(s) to a living body.

### EFFECTS OF THE INVENTION

By the present invention, an antioxidant having higher antioxidant ability than known antioxidative peptides

was provided. Mimosine can be extracted from Leucaena planta flourishing in tropical and subtropical areas of the world. Mimosine is useful as an antioxidant when used as it is alone. In addition, mimosine can potentiate an antioxidant action of a peptide when introduced thereinto, thereby obtaining a peptide with higher antioxidant potency than known antioxidative peptides. A mimosine-containing peptide may be prepared in the form of a short peptide consisting of about 3 to 4 residues, and therefore can be easily synthesized at a low cost. It is known that a peptide antioxidant exhibits synergy with a non-peptide antioxidant and that usage of existing non-peptidic synthetic antioxidants such as butylated hydroxytoluene (BHT) can be largely decreased when a peptide antioxidant is used in combination (Non-patent Document 2). As a novel peptide antioxidant has been provided hereby, the present invention will contribute to decrease of synthetic antioxidant usage. A peptide antioxidant provided by the present invention also has a high tyrosinase inhibitory activity. A mimosine-containing peptide shows higher tyrosinase activity than mimosine used as it is alone, and can exhibit tyrosinase inhibitory activity equally to or higher than kojic acid, a known effective ingredient for skin lightening cosmetics. Therefore, an antioxidant according to the present invention is also useful as an effective ingredient for skin lightening cosmetics.

BRIEF DESCRIPTION OF THE DRAWINGS

Fig. 1 shows the antioxidant ability of the peptide according to the present invention which was examined by DPPH method.
Fig. 2 shows the antioxidant ability of the peptide according to the present invention which was examined by β-carotenc method.
Fig. 3 shows the relationship between the concentration af the respective inhibitors and the tyrosinase activity.
Fig. 4 shows the changes in absorbance over time caused by the antioxidative peptide HPOA05 (Mim-His-Trp-OH) or kojic acid.

MODE FOR CARRYING OUT THE INVENTION

Mimosine (hereinafter also referred to as "Mim" for short) is a compound having the structure shown below and classifed as an amino acid. As shown in the following Examples, evaluation of the antioxidant ability based on the PDDH radical scavenging ability revealed that mimosine has higher antioxidant ability than the known antioxidative tripeptide Pro-His-His. Therefore, mimosine is also useful as an antioxidant when used as it is alone.
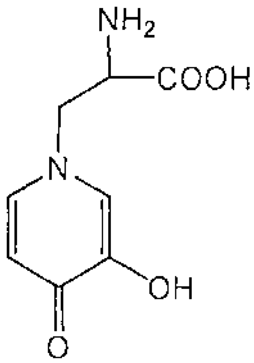

Mimosine is naturally present in Leucaena (scientific name: *Leucaena leucocephala* de Wit), a legume plant flourishing in tropical and subtropical areas. Mimosine is commercially available, and also can be obtained by extraction from Leucaena. For example, mimosine can be obtained by extraction process as follows. Stems and leaves of Leucaena are subjected to a boiling water extraction for about 5 to 20 minutes (about 3 to 7 L of water is used for 1 kg of stems and leaves). The extract may be filtrated as appropriate to remove insoluble substances, and is brought into contact with a strong acid cation-exchange resin (about 800 g to 1500 g per 10 L of extract) so that mimosine is adsorbed on the resin. Thereafter, the resin is washed with water or ethanol of about 80% concentration by immersing the resin thereinto, and then immersed into a basic solvent (such as 1 to 4 N aqueous ammonia) at a volume of about 3 to 7L per 1 kg of resin for about 5 to 7 hours to elute mimosine. After treatment with active carbon as appropriate, the eluent is concentrated, and pH of the eluent is adjusted to 4.5 to 5.0 by adding thereto hydrochloric acid or the like, followed by

keeping it at a low temperature of 0°C to 10°C for about 7 to 24 hours to precipitate mimosine in the solution as crude crystals. The obtained crude crystals are dissolved in aqueous solution of a strong base such as sodium hydroxide, and an acid is added to the resulting solution to adjust its pH to 4.5 to 5.0, followed by leaving it to stand at a low temperature of 0°C to 10°C for about 7 to 24 hours to precipitate high-purity mimosine crystals.

The mimosine-containing peptide is a peptide which comprises at least one mimosine residue in its amino acid sequence. The number of the amino acid residues (including a mimosine residue(s)) of a mimosine-containing peptide is not restricted, and a small sized peptide is preferred from the viewpoint of costs and ease of synthesis. Therefore, the number of the amino acid residues of a mimosine-containing peptide is preferably about 2 to 20, more preferably about 2 to 10, still more preferably about 3 to 4.

As for the mimosine-containing peptide, the sequence of an amino acid residuc(s) other than a mimosine residue(s) is not restricted as long as one or more mimosine residues are contained in its amino acid sequence. As mimosine *per se* has an antioxidant action as it is, it is thought that an antioxidant action is exerted no matter what peptide mimosine may be introduced into. However, from the viewpoint of obtaining a mimosine-containing peptide with a higher antioxidant action, it is preferred to introduce a mimosine residue(s) into a peptide which originally has an antioxidant action. Accordingly, as a mimosine-containing peptide, a peptide consisting of an amino acid sequence which is the same as the amino acid sequence of a peptide originally having an antioxidant action except that at least one amino acid is substituted with mimosine and/or at least one mimosine is added and/or inserted is preferred. By introducing a mimosine residue(s), the antioxidant action a peptide originally has can be further enhanced.

Various antioxidative peptides are known. For example, Non-patent Documents 5 to 12 disclose short peptides having an antioxidant action with a length of about 20 residues or less. Any of such known antioxidative peptides may be used in the present invention. Examples of the known antioxidative peptides are shown in Table 1 below.

Table 1

| PEPTIDE SEQUENCE | ORIGIN |
|---|---|
| Leu-Leu-Pro-His-His (SEQ ID NO: 1) | Enzymatic decomposition product of soybean protein |
| Pro-His-His, Leu-His-Trp, Leu-Trp-Trp, Pro-His-Tyr, Pro-Trp-Trp, Arg-His-Trp, Arg-His-Tyr, Arg-Trp-Trp, His-His-Pro, Leu-Pro-His-His (SEQ ID NO: 2), Tyr-basic amino acid-Tyr (Tyr-Lys-Tyr, Tyr-Arg-Tyr, Tyr-His-Tyr) | Peptide library constructed using enzymatic decomposition product of soybean protein as a lead |
| Ala-His-Lys, Val-His-His, Val-His-His-Ala-Asn-Glu-Asn (SEQ ID NO: 3) | Enzymatic decomposition product of ovalbumin |
| Pro-Ser-His-Asp-Ala-His-Pra-Glu (SEQ ID NO: 4), Val-Asp-His-Asp-His-Pro-Glu (SEQ ID NO: 5), Pro-Lys-Ala-Val-His-Glu (SEQ ID NO: 6), Pro-Ala-Gly-Tyr(SEQ ID NO: 7), Pro-His-His-Ala-Asp-Ser (SEQ ID NO: 8), Val-Asp-Tyr-Pro (SEQ ID NO: 9) | Tuna broth |
| Ala-Arg-His-Pro-His-Pro-His-Leu-Ser-Phe-Met (SEQ ID NO: 10) | Fermented milk |
| Gly-Glu-Hyp-Gly-Pro-Hyp-Gly-Pro-Hyp-Gly-Pro-Hyp-Gly-Prcr-Hyp-G1y (SEQ ID NO: 11), Gly-Pro-Hyp-Gly-Pro-Hyp-Gly-Pro-Hyp-Gly-Pra-Hyp-Gly (SEQ ID NO: 12) | Alaska pollack skin |
| Leu-Met-Ser-Tyr-Met-Trp-Ser-Thr-Ser-Met (SEQ ID NO: 13), Leu-Glu-Leu-His-Lys-Leu-Arg-Ser-Ser-His-Trp-Phe-Ser-Arg-Arg (SEQ ID NO: 14) | Lecithin-free egg yolk |

Among the known antioxidative peptides, it is preferred to introduce a mimosine residue(s) into a tripeptide consisting of 3 amino acids. Especially preferred examples of the antioxidative tripeptide includes those listed below.

Pro-His-His
Tyr-Arg-Tyr
Xaal-His-Tyr (Xaa1 = Pro, Arg, Tyr)
Tyr-Lys-Tyr
Xaa2-His-Trp (Xaa2 = Leu, Arg)

Xaa3-Trp-Trp (Xaa3 = Leu, Pro, Arg)

A site(s) into which a mimosine residue(s) is(are) introduced is(are) not restricted. A mimosine residue(s) may be introduced into the N- or C-terminus of the peptide, or an inner part(s) of the peptide. From the viewpoint of synthesis yield etc., as described below, it is more preferred to introduce a mimosine residue(s) into the N-terminus than the C-terminus. Especially preferred examples of the mimosine-containing peptide includes those listed below.

Mim-Pro-His-His
Mim-Arg-Tyr
Mim-His-Tyr
Mim-Lys-Tyr
Mim-His-Trp
Mim-Trp-Trp
Pro-Mim-His
Pro-His-Mim
Leu-Trp-Mim
Leu-His-Mim

The mimosine-containing peptide has a high tyrosinase inhibitory activity. Although it was already known that mimosine *per se* has a tyrosinase inhibitory activity as it is (Patent Document 1, Non-patent Document 4), as shown in the below-described Examples, evaluation of the tyrosinase inhibitory activity by a known method using tyrosinase and its substrate L-DOPA revealed that mimosine-containing peptides have higher tyrosinase inhibitory activity than mimosine used as it is alone. Therefore, the mimosine-containing peptide is also useful as a tyrosinase inhibitor. Tyrosinase is an enzyme which oxidizes tyrosine to generate melanin and is involved in formation of spots on the skin. A lot of tyrosinase inhibitors such as kojic acid have been traditionally utilized as a skin lightening agent. Therefore, the mimosine-containing peptide is also useful as an ingredient in skin lightening cosmetics and the like.

The antioxidant action of a peptide may be determined by a known method such as DPPH method or β-carotene bleaching method (see, e.g., Chen, H M., Muramoto, K., Yamauchi, F., and Nokihara, K., J. Agric. Food Chem., 44, 2619-2623, 1996. Antioxidant Activity of Designed Peptides Based on the Antioxidative Peptide Isolated from Digests of a Soybean Protein). DPPH method is to measure radical scavenging ability using 1,1-diphenyl-2-picrylhydrazyl (DPPH). If a peptide has an antioxidant action, DPPH having absorption maximum at 517 nm is reduced, and the absorbance at 517 nm decreases. Therefore, a peptide can be determined to have an antioxidant action if the absorbance decreases. In addition, the level of an antioxidant action can be evaluated by the degree of decrease of absorbance. β-carotene bleaching method is to calculate antioxidant ability of an analyte by autoxidizing a mixture of β-carotene, linoleic acid and the analyte and measuring suppression of P-carotene oxidization. If a peptide does not have an antioxidant action, autoxidization proceeds and β-carotene is bleached, which results in decrease of absorbance at 492 nm. Therefore, if the absorbance does not decrease, the peptide can be determined to have an antioxidant action. In addition, the level of an antioxidant action can be evaluated by the degree of decrease of absorbance. Such known evaluation methods as described above reflect a part of antioxidant mechanisms which act in a living body in parallel. Thus, a given peptide can be considered to be useful as an antioxidant if any one of the evaluation methods confirms that the peptide has an antioxidant action.

Tyrosinase inhibitory activity of a peptide can be evaluated by a known method using tyrosinase and its substrate such as L-dihydroxyphenylalanine (L-DOPA) or L-tyrosine. Specific examples of the evaluation method are described in the Examples below. For example, a peptide whose tyrosinase inhibitory activity should be measured, tyrosinase, and its substrate are dissolved in phosphate buffer, respectively (the peptide and tyrosinase may be dissolved in the same solution). Immediately after mixing the three components, change in absorbance at 475 nm is measured with a spectrophotometer. Since melanin has an absorption maximum at 475 nm, absorbance at 475 nm increases if melanin is synthesized from L-DOPA by tyrosinase. If the tyrosinase activity is inhibited by the peptide, elevation of absorbance at 475 nm is suppressed. Therefore, the tyrosinase inhibitory activity of a peptide can be evaluated based on the change in absorbance.

Synthesis of the mimosine-containing peptide can be easily performed using conventional solid-phase peptide synthesis technique. As for a C-terminal amino acid used in solid-phase method, solid-phase resins to which a C-terminal amino acid of a peptide of interest is bound are commercially available, and such a commercial produet may be preferably used. For example, a polystyrene resin having a linker (cleavable by trifluoroacetic acid (TFA)) developed by Wang, or a chlorotrityl-resin which allows even a weak acid to cleave the binding between the peptide chain and the resin may be preferably used. Mim can also be arranged in the C-terminus of a peptide chain by a method commonly used in peptide synthesis. In this case, Mim is introduced by a common method into a carrier (resin) on which a linker cleavable under the condition employed in conventional solid-phase peptide synthesis is arranged. Amino acids are bound to the Mim resin one by one to obtain a protected peptide resin, and the protected peptide resin is subjected to cleavage so that a mimosine-containing peptide can be obtained. However, from the viewpoint of the yield and the like, it is more advantageous to introduce Mim into the N-terminus because costs is lower and purification can be conducted easily

thereby.

A fluorenylmethoxycarbonyl group (Fmoc group) may be used for protection of the Na-amino groups, and piperidine solution, which is used in a conventional Fmoc method, may be used for removal of the Fmoc group. Protection of the amino group of a mimosine molecule may also be performed by the Fmoc method. For example, Fmoc protection reaction may be preferably carried out in an aqueous solvent such as a mixed solvent of water:dioxane = 1:1 using 2 to 5 equivalents of base and 1 to 3 equivalents of protecting reagent (e.g. Fmoc-OSu) with respect to mimosine. The amount of mimosine added to the reaction system is usually about 15 to 60 g/L.

For the side chain protection, a series of protecting groups conventionally used for the Fmoc-tBu type, i,e., a *tert*-butoxycarbonyl group (Boc group) for amino groups (Lys, Trp), a triphenylmethyl group (Trt group) for an imidazole group of His, and 2,2,4,6,7-pentamethoxytdihydrobenzofuran-5-sulfonyt group (Pbf group) for a guanidino group of Arg, may be used. These groups are preferred as they can be easily removed by TFA in the cleavage step. The methods *per se* for protecting the functional groups in the side chains of amino acids are well-known (e.g., Kiyoshi NOKIHARA, Journal of Synthetic Organic Chemistry Japan, 52, 347-358, 1994, "Highly Efficient Peptide Synthesis: Simultaneous and Automatic Synthesis of Multiple Peptides and Peptide Library"; Kiyoshi NOKIHARA and Yoshio YOKOMIZO, Shimadzu Review, 52, 1-8, 1995, "Cleaving Off of Synthetic Peptides by Fmoc-tBu Strategy"; Kiyoshi NOKIHARA, Shimadzu Review, 50, 13-24, 1993, "Reagents for Highly Efficient Solid Phase Synthesis of Pcptides"; Kiyoshi NOKIHARA, Shimadzu Review, 50, 3-12, 1993, "Fundamental of Peptide Chemical Synthesis"; K. Nokihara, R. Yamamoto, M. Hazama. O. Wakizawa, S. Nakamura, and M. Yamaguchi, Peptide Chemistry 1991, ed., A. Suzuki, Protein Research Foundation, Osaka, 203-208., 1992, "Development and Applications of a Novel and Practical Simultaneous Multiple Solid Phase Peptide Synthesizer", Chan, W. C.; White, P. D. Fmoc Solid Phase Peptide Synthesis: A Practical Approach; Oxford University Press: New York, 2000; pp 41- 76.).

Introduction of each protected amino acid and protected mimosine (acyl components) may be carried out by activating carboxyl groups of the acryl components with benzotriazol-1-yl-oxy-tris(pyrrolidino)phosphonium hexafluorophosphate (PyBOP), 1-hydroxylbenzotriazole (HOBt) in the presence of a base and then reacting them with an amino component.

A protected peptide-resin may be obtained by introducing all of the amino acids of interest. Nα-Fmoc contained in the protected peptide-resin may be removed in accordance with a conventional method. After washing the resin with DMF, methanol, *t*-butylmethyl ether, and drying it thoroughly, cleavage may be carried out using TFA to obtain a mimosine-containing peptide which comprises a mimosine residue(s) introduced into a desired site. In this process, TFA mixed with scavenger reagents for trap of secondary generated cations is preferably used. This mixture may be called as "cleavage cocktail" in this field, and it is known that byproducts can be greatly decreased by using the cocktail. If desired, the molecular weight etc. of the synthesized peptide may be checked by ESI-Ion-trap-MS (electrospray ionization mass spectrometry), Prior to the ESI-Ion-trap-MS, the peptide obtained after cleavage may be precipitated with TFA/diethylether, purified by reverse phase HPLC, and subjected to reverse phase HPLC to confirm that the peptide consists of a single component.

The antioxidant according to the present invention comprises mimosine and/or a mimosine-containing peptide (s) as described above. The antioxidant may comprise both mimosine and a mimosine-containing peptide(s). As for the mimosine-containing peptide, the antioxidant may comprise two or more mimosine-containing peptides. The antioxidant may comprise no other ingredients besides mimosine and/or a mimosine-containing peptide(s), or may comprise other ingredient(s) useful for stabilizing these effective ingredients. The antioxidant may further comprise other known antioxidative peptide(s) and/or a known non-peptide antioxidant(s) (such as butylated hydroxytoluene (BHT), α-tocopherol and the like). It is also known that usage of existing non-peptidic synthetic antioxidants can be largely reduced when a peptide antioxidant is used in combination (Non-patent Document 2).

The mimosine-containing peptide has a higher tyrosinase inhibitory activity than mimosine as it is alone. Hence, the mimosine-containing peptide is also useful as a tyrosinase inhibitor. Tyrosinase is an enzyme which oxidizes tyrosine to generate melanin and is involved in formation of spots on the skin. Therefore, the mimosine-containing peptide is also useful as an effective ingredient in a composition for skin lightening. The composition for skin lightening may be used as a material for skin care cosmetics such as a facial wash, a face lotion, a milky lotion, a beauty essence, etc., and make up cosmetics such as foundation. It is also known that certain peptides can be used to give functions and/or effects to a cosmetic and that such a functionalization is sufficiently achieved at a peptide concentration of just several to 10 ppm in a cosmetic. In the case where the mimosine-containing peptide(s) is(are) added to a cosmetic, the peptide concentration may be several to 10 ppm. However, the concentration is not restricted thereto, and may be appropriately determined.

The antioxidant according to the present invention may be incorporated into articles such as foods, cosmetics, pharmaceuticals etc. so that oxidation of ingredients in the articles can be suppressed and thus the preservative quality of the articles can be improved. In addition, it was recently reported that active oxygen is associated with cancer, stroke, aging, various lifestylc-related diseases etc., and hence functional molecules having an antioxidant ability began to be focused. The antioxidant according to the present invention can remove active oxygen in a living body by being administered thereto, and therefore is useful for prevention of cancer, stroke, aging, various lifestyle-related diseases etc. The antioxidant of the present invention is also useful for beauty, for example, prevention of wrinkling and spotting and the like. Administration route is not restricted, and the antioxidant of the present invention may be administered parenterally or orally, or systemically or topically. The antioxidant of the present invention may be added to food products so that nutraceuticals for antioxidation can be provided.

According to the present invention, the antioxidant action which known antioxidative peptides originally have can be improved by introducing thereinto a mimosine residue(s). Hence, the present invention also provides a method for improving an antioxidants action of a peptide. The method comprises substituting at least one amino acid in a peptide having an antioxidant action with mimosine; adding at least one mimosine to the peptide; and/or inserting at least one mimosine into the peptide. The phrase that substituting at least one amino acid with mimosine, adding mimosine and/or inserting mimosine, includes to synthesize a peptide whose sequence comprises substitution with mimosine etc. in a manner mentioned. Peptides having an antioxidant action are known as described above, and conditions for the preferred antioxidative peptide are same as above.

A peptide containing a mimosine residue(s) introduced thereinto in accordance with the above-described procedures has a higher antioxidant action than the peptide before mimosine introduction. Whether the antioxidant action of a peptide is improved or not may be evaluated by a known technique such as DPPH method or β-carotene bleaching method described above.

EXAMPLE

The present invention will now be described more concretely by way of an example thereof. However, the present invention is not restricted to the example below.

1. Acquisition of Purified Mimosine

Mimosine was extracted from stems and leaves of Leucaena in the following manner. In 100 l. of boiling water, 25 kg of fresh Leucaena leaves were extracted for 10 minutes. About 100 L of the extract was cooled to room temperature, and insoluble substances were removed by filtration. Thereafter, 10 kg of strong acid cation exchange resin (AMBERLITE (trademark) IR120 H(Plus), GFS Chemicals Inc., Ohio, U.S.) was added to the extract, and mimosine was adsorbed overnight. The ion exchange resin was washed by immersion in 25 l of 80% ethanol for 12 hours and then in 25 L of distillled water for 2 hours. The ion exchange resin was immersed in 40 L of 2N aqueous ammonia for 6 hours to elute mimosine from the ion exchange resin. The eluate was firstly treated with active carbon. In the present reaction scale, 5 g of active carbon was used. The mixture was stirred for 10 minutes at room temperature, and active carbon was removed by filtration. The obtained filtrate was concentrated under reduced pressure. The concentrated liquid was adjusted to pH 4.5 to 5.0 with 6N HC1, and kept at 4°C overnight, thereby precipitating crude mimosine hydrochloride to obtain crude mimosine crystals (melting point 175°C with decomposition). The crude crystals were recovered by filtration and dissolved in 1.8 L of 6N NaOH aqueous solution. After neutralization with 6N HCl, the solution was kept at 4°C overnight, thereby recrystallizing mimosine to obtain 100 g of mimosine hydrochloride crystals.

2. Synthesis of Mimosine-Introduced Peptide

(1) Synthetic peptides containing Mim at the site other than C-terminus

All the synthetic peptides were synthesized by conventional solid-phase peptide synthesis technique using PetiSyzer (tradename), the personal multiple synthesizer manufactured by HiPep Laboratories, The concrete procedures were as follows. As a C-terminal amino acid, a solid-phase resin (commercial product) on which a C-teriminal amino acid of a peptide of interest was immobilized was used. As a resin, a polystyrene resin having a linker (cleavable by trifluoroacetic acid (TFA)) developed by Wang, or a chlorotrityl resin which allowed even a weak acid to cleave the binding between the peptide chain and the resin was used. A fluorenylmethoxycarbonyl group (Fmoc group) was used for protection of the Nα-amino group of amino acids and mimosine, and a solution of 2% piperidine and 2% 1,8-diazabicyclo-[5,4,0]-7- undecene (DBU) in DMF, which was used in conventional Fmoc method, was used for removal of Fmoc protecting group. For the side chain protection, those conventionally used for protection of the Fmoc-tBu type, that is, a *tert*-butoxycarbonyl group (Boc group) for amino groups (Lys, Trp), a triphenylmethyl group (Trt group) for an imidazole group of His, and 2,2,4,6,7-pentamethoxyldihydrobenzofuran-5-sulfonyl group (Pbf group) for a guanidino group of Arg, were used. These could be easily removed with TFA in cleavage step. Coupling of the protected amino acids and mimosine (acyl components) was carried out by activating carboxyl groups with benzotriazal-1-yl-oxy-tris(pyrrolidino) phosphonium hexafluorophosphate (PyBOP) and 1-hydroxylbenzotriazole (HOBt) in the presence of a base and binding the carboxyl groups to the amino groups of an amino component. After introducing all of the amino acids of interest, the

Na-Fmoc present in the obtained protected peptide-resin was removed with a base in accordance with a common method. The resin was washed with DMF, methanol and *t*-butylmethyl ether and dried fully, followed by cleavage with TFA to separate the peptide from the resin. As a cleavage solution, a cocktail TFA/TIS (triisopropylsilane)/water = 90/5/5 was used, and a cleavage cocktail to which 2-methylindole was added was used for peptides comprising a Trp residue(s).

All of the synthesized peptides were precipitated with TFA/diethyl ether after the cleavage, dissolved in 0.1% TFA or 10 mM hydrochloric acid-30% acetonitrile, and then freeze-dried. Each peptide was used in the antioxidation assays after confirming that the peptide consisted of a single component by LCMS using a reverse phase HPLC column (electrospray ionization mass spectrometry [ESI-Ion-trap-MS]).

The reverse phase HPLC was carried out using HiPep-CadenzaC18-3015S (3.0 x 150 mm) manufactured by HiPep Laboratories. The flow rate was 0.5 ml/min, and the detection was performed by UV method (210 run). The elution was carried out for 30 minutes by a linear concentration gradient as follows: using 0.1% TFA as solvent A and 0.1% TFA-90% acetonitrile as solvent B, A/B was changed from 99/0] to 69/31 (elution time Rt = (a)); from 85/15 to 55/45 (elution time Rt = (b)); from 85/15 to 55/45 (elution time Rt - (c)); or from 80/20 to 50/50 (elution time Rt = (d)). ESIMS was measured with a mass spectroscope manufactured by Bruker Daltonics Inc.

(2) Synthesis of Resin for Arranging Mimosine Residue in C-terminus: Mim-2-Cl-Trt-Resin Synthesis

Dried dichloromethane (DCM) and diisopropylethylamine (DIEA) were desiccated overnight by molecular sieves. Twenty grams of Cl-(2-Cl-Trt)resin (0.98 mmol/g) (NovaBioChem, P/N:01-64-0114) and 8.4 g of Fmoc-Mim-OH (HiPep Laboratories, Inc.) were weighed and added to an Erlenmeyer flask and a recovery flask, respectively, and vacuum-dried overnight in a desiccator containing diphosphorus pentoxide. DCM (100 mL) and DIEA (13.7 mL, 80 mmol) were added to the recovery flask containing Fmoc-Mim-OH to dissolved Fmoc-Mim-OH. Under a nitrogen atmosphere, ice-cold Fmoc-Mim-OH solution was added dropwise to an Erlenmeyer flask containing a resin. Paying attention to generation of heat and smoke, the total amount of the reaction solution was added while repeating stirring and ice-cooling. The resulting solution was cooled to room temperature, and manually stirred at 5- to 10-minute intervals to proceed reaction for 60 minutes. After completion of the reaction, the resin was recovered in a glass filter by filtration, and washed three times with a solution of DCM: methanol (MeOH):DIEA=17:2:1, three times with DCM, and then three times with dimethylformamide (DMF), each time using 100 mL of the wash solvent. For calculation of the substitution rate, about 10 mg of the resin wetted with DMF was weighed, washed 3 times with methanol and then 3 times with *t*-butymethyl ether (tBME), and vacuum-dried for about 1 hour. After measuring the accurate weight of the resin, 0.5 mL of the piperidine(PIP):DCM=1:1 solution was added thereto to carry out Fmoc deprotection reaction for 10 minutes. The reaction solution was transferred to a 50 mL volume measuring flask through filtration. The resin was washed with DCM (1 mL x 3), and the washings were also added to the measuring flask. The volume was adjusted to 50 mL with DCM, and after stirring thoroughly, UV measurement was performed (30 nm, 290 nm, 267 nm). The substitution rate was calculated according to the following formulae, and the intermediate value was adopted.

301 nm: Substitution Rate = {(A/1.56/resin mg}x10
290 nm: Substitution Rate = {(A/1.16/resin mg)x10
267 nm: Substitution Rate = {(A/3.5/resin mg}x10
Substitution Rate of Mim-2-Cl-Trt-Resin: 0.45 mmol/g

After confirming the substitution rate, Fmoc deprotection of the resin was carried out with PIP:DBU:DMF = 2: 2:96. After Fmoc deprotection, the resin was washed 3 times with DCM, 3 times with MeOH and 3 times with tBME, each time using 100 mL of the wash solvent. After washing, the resin held in the glass filter was placed as it was in a desiccator for 2-3 days to dry the resin under reduced pressure, and the weight was measured (yield of Mim-2-Cl-Trt-Resin: 26.8 g). Unless otherwise specified, the reagents used herein were those manufactured by Nacalai Tesque, inc.

Solid-phase synthesis was carried out in the same manner as in (1) above except that Mim-2-Cl-Trt-Resin prepared as described above was used to synthesize mimosine-introduced peptides comprising mimosine in the C-terminus.

Table 2

| Sample Name* (X is a control peptide not containing Mim) | Amino Acid Sequence of Synthetic Peptide | HPLCRt (min)** | | Measured Value $[M+H]^+$ | M=Molecular Weight Theoretical Value |
|---|---|---|---|---|---|
| 1 | Mim-Pro-His-His | 8.2 | (a) | 570.3 | 570.6 |
| 1X | Pro-His-His | 6.3 | (a) | 390.3 | 390.4 |
| 2 | Mim-Arg-Tyr | 14.8 | (a) | 518.3 | 518.5 |

(continued)

| Sample Name* (X is a control peptide not containing Mim) | Amino Acid Sequence of Synthetic Peptide | HPLCRt (min)** | | Measured Value $[M+H]^+$ | M=Molecular Weight Theoretical Value |
|---|---|---|---|---|---|
| 2X | Tyr-Arg-Tyr | 19.1 | (a) | 501.3 | 501.6 |
| 3 | Mim-His-Tyr | 14.2 | (a) | 499.3 | 499.5 |
| 3X | Pro-His-Tyr | 15.2 | (a) | 416.2 | 416.5 |
| 4 | MimLys-Tyr | 14.2 | (a) | 490.3 | 490.5 |
| 4X | Tyr-I,ys-Tyr | 18.4 | (a) | 473.3 | 473.5 |
| 5 | Mim-His-Trp | 20.9 | (a) | 522.3 | 522.5 |
| 5X | Arg-His-Trp | 19.7 | (a) | 498.3 | 498.6 |
| 6 | Mim-Trp-Trp | 18.3 | (c) | 571.3 | 571.6 |
| 6X | Leu-Trp-Trp | 19.3 | (d) | 504.3 | 504.1 |
| 7 | Pro-Mim-His | 6.8 | (a) | 433.3 | 433.4 |
| 8 | Lcu-His-Mim | 10.5 | (a) | 449.3 | 449.5 |
| 8X | Leu-His-Trp | 19.6 | (b) | 455.2 | 455.5 |
| 9 | Leu-Trp-Mim | 13.9 | (b) | 498.3 | 498.6 |
| 10 | Pro-His-Mim | 6.6 | (a) | 433.2 | 433.4 |

*Samples 7 and 10 were obtained by substitution of Sample 1X with mimosine; Sample 9 was obtained by substitution of Sample 6X with mimosine.
**Separation Condition in HPLC (linear AB gradient for 30 min)
Solvent A: 0.1% TFA, Solvent B: 0.1% TFA-90% acetonitrile
(a) A/B = 99/01 - 69/31
(b) A/B = 90/10 - 60/40
(c) A/B = 85/15-55/45
(d) A/B = 80/20 - 50/50

3. Acute Dermal Toxicity Study of Mimosine and Mimosine-Introduced Peptides: Preparation of Mimosine Cream

Vegetable emulsified wax (emulsifying wax. Orangeflower Inc., ingredients: stearyl alcohol, polysorbate 60) and oil (edible olive oil, Ajinomoto Co., Inc.) were completely melted in hot water (50°C). Warm water (ultrapure water) was added thereto, and the mixture was mixed. An aqueous solution of mimosine or mimosine-introduced peptide was added thereto, and the mixture was completely mixed up. The obtained mixture was stored at 4°C until use in the animal experiment.

The left and right sides of the trunk of ddY mice (9-week-old, male, 6 mice in each group) were shaved with clippers under anesthesia with ether. The cream in which mimosine or mimosine-introduced peptide was mixed was applied to the both sides twice a day for three days, and the skin condition was observed including during the test. Any special changes were not observed. The mice were anatomized at day 5 and their skins were carefully surveyed, which revealed that there were no difference between the skins of control mice (to which a cream not containing mimosine nor a mimosine-introduced peptide was applied) and those tested. It was confirmed that neither mimosine nor a mimosine-introduced peptide has an acute dermal toxicity. Therefore, it is thought that mimosine and mimosine-introduced peptide can be utilized in the cosmetic field.

4. Measurement of Antioxidant Activity

(1) DPPH Radical Scavenging Ability

The mimosine-introduced peptides synthesized above were dissolved in ethanol to prepare 1 mL of 1 mM peptide solutions, respectively. Similarly, peptides which were already known to have an antioxidant action but did not comprise Mim, such as the antioxidative peptide (Pro-His-His), and BHT as a positive control were dissolved to prepare 1 mL of 1 mM solutions, respectively, which were used as an undiluted solution for measurement. Next, DPPH was dissolved in ethanol to prepare 10 mL of 0.1 mM DPPH-cthanol solution, and 0.15 mL of this DPPH-cthanol solution and 0.15 mL of the above-prepared undiluted solution were mixed and stirred (the sample concentration: 500 μM). The reaction was allowed to proceed for 50 minutes at room temperature, and the absorbance at 517 nm wavelength was measured with a UV-VIS spectrophotometer (JASCO V-570). For blanks, a blank solution was prepared using the same

volume of ethanol instead of DPPH, and the absorbance of the blank solution was measured via the same reaction condition. For controls, a control solution was prepared using ethanol instead of sample solutions, and the absorbance of the control solution was measured via the same reaction condition.

The DPPH radical scavenging ability was calculated according to the following formula.

$$\text{Radical Scavenging Ability} \quad \text{AntOx}(\%) = (\text{Acon} - \text{Asam}) / \text{Acon} \times 100$$

Acon = The absorbance of the control after being left to stand for 50 minutes.
Asam = The absorbance of the sample after being left to stand for 50 minutes.

The BHT-rclative antioxidant ability was calculated according to the following formula.

$$\text{BHT-Relative Antioxidant Ability} \quad (\%) = [\text{AntOx}(\%)] / [500\mu M\ \text{BHT}(\%)] \times 100$$

The results are shown in Table 3 and Fig. 1. The BHT-relative radical scavenging ability (antioxidant potency) of the antioxidative peptide which was used as a positive control was 23.01%. In the case where mimosine was introduced thereinto, the antioxidant ability increased to 111.67%, which was about 5-fold higher than the original peptide. All of the peptides according to the present invention showed a radical scavenging ability much higher than the known antioxidative peptide Pro-His-His. Especially, Samples 1, 5 and 6 showed a radical scavenging ability at the same level as or higher than BHT, an antioxidant generally used.

Table 3

| Sample Name | Amino Acid Sequence | BHT-Relative Antioxidant Ability (%) DPPH Method |
|---|---|---|
| BHT | | 100.00 |
| Mimosine | | 81.32 |
| 1X | Pro-His-His | 23.01 |
| 1 | Mim-Pro-His-His | 111.67 |
| 7 | Pro-Mim-His | 37.04 |
| 10 | Pro-His-Mim | 26.45 |
| 2X | Tyr-Arg-Tyr | 11.31 |
| 2 | Mim-Arg-Tyr | 77.89 |
| 3X | Pro-His-Tyr | 9.63 |
| 3 | Mim-Ilis-Tyr | 84.88 |
| 4X | Tyr-Lys-Tyr | 9.55 |
| 4 | Mim-Lys-Tyr | 75.33 |
| 5X | Arg-His-Trp | 11.26 |
| 5 | Mim-His-Trp | 99.06 |
| 6X | Lcu-Trp-Trp | 73.27 |
| 6 | Mim-Trp-Trp | 117.34 |
| 9 | Leu-Trp-Mim | 41.02 |
| 8X | Leu-His-Trp | 41.92 |
| 8 | Leu-His-Mim | 26.45 |

(2) Measurement of β-Carotcne Bleaching Ability

β-carotene was dissolved in chloroform to prepare 1 mg/mL solution. Linoleic acid was dissolved in chloroform to prepare 0.1 g/mL solution. Tween 40 was dissolved in chloroform to prepare 0.2 g/mL solution. Into 100 mL recovery flasks, 0.15 mL of β-carotene solution in chloroform, 0.1 mL of linoleic acid solution in chloroform, and 0.3 mL of Tween 40 solution in chloroform were aliquoted, and the mixed solution was evaporated to dryness with an evaporator until the smell of chloroform was completely disappcared. The dried mixture was dissolved in 30 mL of pure water, and 2.7 mL of 0.2 M phosphate buffer (pH 6.8) was further added thereto, followed by gentle stirring to prepare carotene-linoleic acid solution. Next, the peptides obtained in Examples 1 were dissolved in ethanol to prepare 1 mL of 0.05 mM peptide solutions, respectively. Similarly, peptides which were already known to have an antioxidant action but did not comprise Mim, such as the antioxidative peptide (Pro-His-His), and BHT as a positive control were dissolved to prepare 1 mL of 0.05 mM solutions, respectively, which were used as an undiluted solution for measurement.

Thereafter, 0.45 mL of carotene-linoleic acid solution and 0.05 mL of undiluted sample solution were mixed and stirred (sample concentration: 5 μM), and the reaction was allowed to proceed for 50 minutes at 50°C. Five minutes and 50 minutes after the start of the reaction, the absorbance at OD 492 nm was measured with a UV-VIS spectrophotometer. For blanks, a blank solution was prepared using the same volume of distilled water instead of carotene-linoleic acid solution, and the absorbance of the blank solution was measured via the same reaction condition. For controls, a control solution was prepared using ethanol instead of sample solutions, and the absorbance of the control solution was measured via the same reaction condition.

The antioxidant ability was calculated according to the following formula.

$$\text{Antioxidant ability} \quad (\%) = [\ 1 - (\ A5S - A50S\ )\ /\ (\ A5C - A50C\ )\ ]\ x100$$

A5C = The absorbance of the control after 5-minute reaction.
A5S = The absorbance of the sample after 5-minute reaction.
A50C = The absorbance of the control after 50-minute reaction.
A50S = The absorbance of the sample after 50-minute reaction.

The BHT-relative antioxidant ability was calculated according to the fallowing formula.

$$\text{BHT-Rclative Antioxidant Ability} \quad (\%) = [\text{AntOx}(\%)]\ /\ [5\mu\text{M BHT}(\%)]\text{x}100$$

The results are shown in Table 4 and Fig. 2. The antioxidant potency of the antioxidative peptide which was measured as a positive control was 63.00%. In the case where mimosine was introduced thereinto, the antioxidant potency increased to 93.86%, which was about 1.5-fold higher than the original peptide. All the peptides according to the present invention showed an antioxidant ability higher than the known antioxidative peptide Pro-His-His. It was confirmed that Samples 1, 3 and 5 have an antioxidant ability at the same level as BHT, an antioxidant generally used. In this evaluation by β-carotene method, the antioxidant potency of mimosine molecule alone was no more than 15.41%, and that of the mimosine-introduced peptide was as high as 93.86%, which was about 6-fold higher than the mimosine molecule alone.

Table 4

| Sample Name | Amino Acid Sequence | BHT-Relative Antioxidant Ability (%) β-Carotene Method |
|---|---|---|
| BHT | | 100.00 |
| Mimosine | | 15.41 |
| 1X | Pro-His-His | 63.00 |
| 1 | Mim-Pro-His-His | 93.86 |
| 7 | Pro-Mim-His | 73.07 |
| 10 | Pro-His-Mim | 79.55 |
| 2X | Tyr-Arg-Tyr | 7.78 |

(continued)

| Sample Name | Amino Acid Sequence | BHT-Relative Antioxidant Ability (%) β-Carotene Method |
|---|---|---|
| 2 | Mim-Arg-Tyr | 66.77 |
| 3X | Pro-His-Tyr | 33.56 |
| 3 | Mim-His-Tyr | 95.80 |
| 4X | Tyr-Lys-Tyr | 18.88 |
| 4 | Mim-Lys-Tyr | 81.96 |
| 5X | Arg-His-Trp | 41.37 |
| 5 | Mim-His-Trp | 92.66 |
| 6X | Leu-Trp-Trp | 28.28 |
| 6 | Mim-Trp-Trp | 72.56 |
| 9 | Leu-Trp-Mim | 77.32 |
| 8X | Lcu-His-Trp | 46.79 |
| 8 | Leu-His-Mim | 77.79 |

5. Measurement of Tyrosinase Inhibitory Activity

(1) Materials

Mushroom tyrosinase (SIGMA-Aldrich, Missouri, USA)
L-DOPA (SIGMA-Aldrich, Missouri, USA)
50 mM Sodium phosphate buffer (pH6.8)
Mimosine (Lot. 20070929)
Kojic acid (Wako, Japan)
Antioxidative peptides (Synthetic products HPOA01, 03, 05, 06 manufactured by HiPep Laboratories. Sequences arc shown in Table 5.)

(2) Method

(i) Measurement of Tyrosinase Activity

Using 50 mM sodium phosphate buffer (pH 6.8), 5 mM L-DOPA solution, mushroom tyrosinase solution (2 units/μL) and 1 mM inhibitor stock solution (x10) were prepared. Furthermore, the stock solutions at a concentration of 0.5, 0.1, 0.05, 0.01 mM, and 0.005 mM (only in the case of HPOA05 and kojic acid) were prepared by diluting 1mM stock solution.

Reaction Solution A (25 μL of 5 mM DOPA solution; 75 μL of 50 mM sodium phosphate buffer (pH 6.8)) and Reaction Solution B (25 μL of inhibitor stock solution; 12.5 μL of tyrosinase solution; 112.5 μL of 50 mM sodium phosphate buffer (pH 6.8)) were prepared in two Eppendorf tubes, respectively. Reaction Solutions A and B were mixed just before the measurement, and the absorbance at 475 nm wavelength was measured with a spectrophotometer (V-570, Jasco, Tokyo, Japan). The measurement was carried out for 60 seconds at room temperature, and the absorbance was recorded every 1 second. This operation was repeated 3 times for each sample concentration.

(ii) Calculation of $IC_{50}$

The tyrosinase activity ratio at each inhibitor concentration was calculated based on the control (tyrosinase alone) value, and $IC_{50}$s for tyrosinase inhibition of the samples were calculated using Ngraph. The fitting formula below was used in the calculation.

$$y=a-(a-b)/(1+(x/c)^d$$

(a= Minimum value of y; b = Maximum value of y; c = Inhibitor concentration; d = Variable)

(3) Results

In this experimental condition, $IC_{50}$s of mimosine and kojic acid measured as a control were 38.3 and 6.49 μM, respectively. On the other hand, $IC_{50}$s of HPOA01, 03, 05 and 06 were 16.4, 8.84, 3.02 and 28.2 μM, respectively (Table 5, Fig. 3). All the antioxidative peptides showed a tyrosinase inhibitory activity higher than mimosine, and the inhibitory activity varied depending on the amino acid sequence. In particular, HPOA05 (Mim-His-Trp-OH, Mwt: 598.50) showed a tyrosinase inhibitory activity higher than kojic acid. These results suggest that the antioxidative peptides (especially HPOA05) act as a potent inhibitor in the catalytic oxidation reaction of L-DOPA by tyrosinase.

The mechanism of inhibition is unclear. In the case of antioxidative peptides, the reaction velocity (changes in absorbance) at a high concentration range gradually decreased over time, whereas, in the case of kojic acid, the reaction velocity at a low concentration range slightly increased over time (Fig. 4), which suggests that the mechanism varies depending on the types of the inhibitors (e.g., kojic acid acts on tyrosinase more directly, while antioxidative peptides act on tyrosinase indirectly (e.g. on L-DOPA)).

Table 5

| Sample Name (Inhibitor) | $IC_{50}$ (μM) | Amino Acid Sequence |
|---|---|---|
| HPOA01 | 16.4 | Mim-Pro-His-His |
| HPOA03 | 8.84 | Mim-His-Tyr-OH |
| HPOA05 | 3.02 | Mim-His-Trp-OH |
| HPOA06 | 28.2 | Mim-Trp-Trp-OH |
| Kojic acid | 6.49 | |
| Mimosine | 38.3 | |

## Claims

1. An antioxidant comprising mimosine and/or a mimosine-containing peptide(s).

2. The antioxidant according to claim 1, wherein said mimosine-containing peptide(s) each comprises at least one mimosine residue in the amino acid sequence.

3. The antioxidant according to claim 1 or 2, wherein the number of the amino acid residues of said mimosine-containing peptide(s) is 2 to 20.

4. The antioxidant according to claim 3, wherein the number of the amino acid residues of said mimosine-containing peptide(s) is 3 or 4.

5. The antioxidant according to any one of claims 1 to 4, wherein said mimosine-containing peptide(s) each consists of the same amino acid sequence as that of a peptide having an antioxidant action except that at least one amino acid is substituted with mimosine and/or at least one mimosine is added and/or inserted.

6. The antioxidant according to claim 5, wherein said peptide having an antioxidant action is any one of the following peptides.
Pro-His-His
Tyr-Arg-Tyr
Xaa1-His-Tyr (Xaa1 = Pro, Arg, Tyr)
Tyr-Lys-Tyr
Xaa2-His-Trp (Xaa2 = Leu, Arg)
Xaa3-Trp-Trp (Xaa3 = Leu, Pro, Arg)

7. The antioxidant according to claim 6, wherein said mimosine-containing peptide(s) each is represented by any one of the following structures.
Mim-Pro-His-His
Mim-Arg-Tyr
Mim-His-Tyr
Mim-Lys-Tyr
Mim-His-Trp
Mim-Trp-Trp
Pro-Mim-His
Pro-His-Mim
Leu-Trp-Mim
Leu-His-Mim
(Mim represents a mimosine residue)

8. A method for improving an antioxidant action of a peptide, comprising substituting at least one amino acid in the peptide with mimosine, adding at least one mimosine to the peptide, and/or inserting at least one mimosine into the peptide.

9. A tyrosinase inhibitor comprising a mimosine-containing peptide(s).

10. The tyrosinase inhibitor according to claim 9, wherein said mimosine-containing peptide(s) each is represented by any one of the following structures.
Mim-Pro-His-His
Mim-His-Tyr
Mim-His-Trp
Mim-Trp-Trp
(Mim represents a mimosine residue)

11. A composition for skin-lightening comprising as an effective ingredient the tyrosinase inhibitor according to claim 9 or 10.

12. Mimosine or a mimosine-containing peptide for use in antioxidation.

13. A mimosine-containing peptide for use in tyrosinase inhibition.

14. The mimosine-containing peptide according to claim 13 for use in skin-lightening.

**15.** A method for antioxidation of an article, comprising incorporating mimosine and/or a mimosine-containing peptide (s) into an article in which antioxidation is desired.

**16.** A method for removing active oxygen in a living body, comprising administering mimosine and/or a mimosine-containing peptide(s) to a living body.

**17.** A method for inhibiting tyrosinase in a living body, comprising administering a mimosine-containing peptide(s) to a living body.

**18.** The method according to claim 17, which is for skin-lightening.

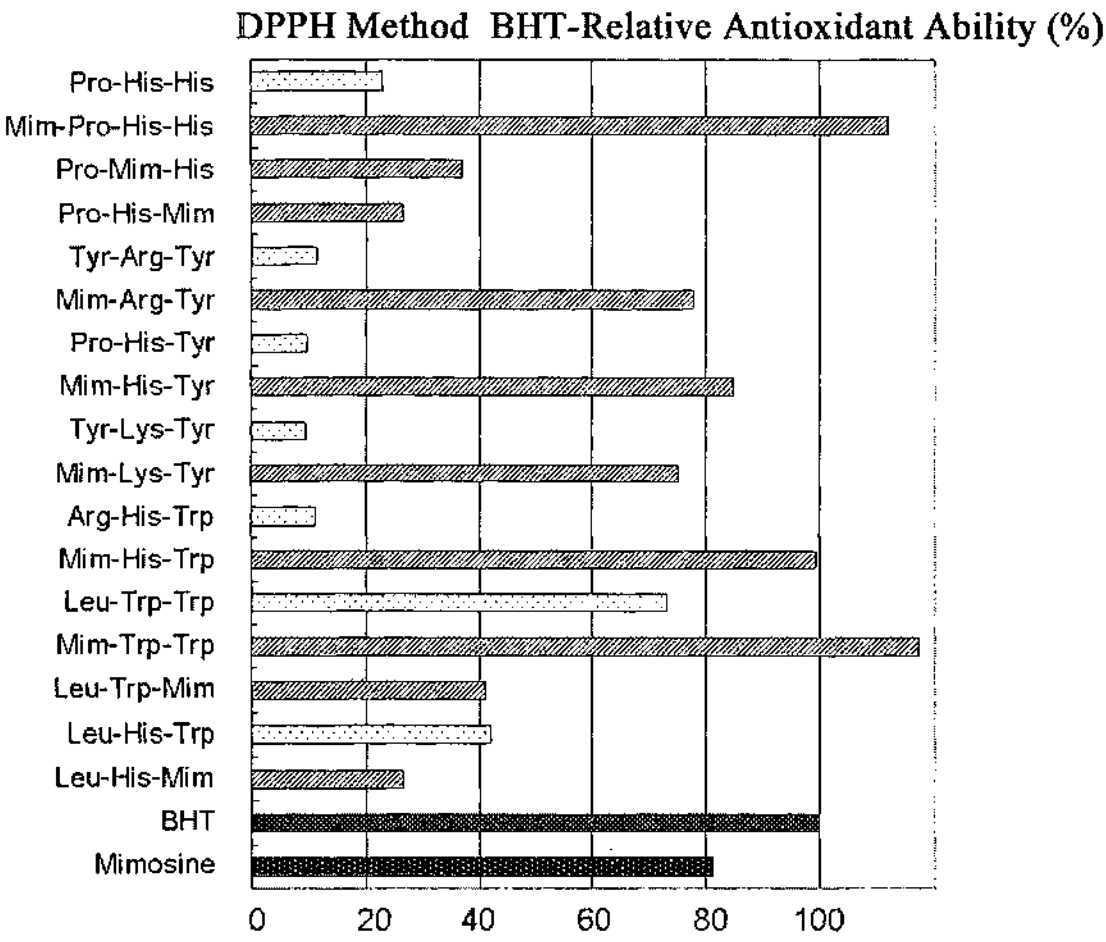
DPPH Method BHT-Relative Antioxidant Ability (%)
Pro-His-His
Mim-Pro-His-His
Pro-Mim-His
Pro-His-Mim
Tyr-Arg-Tyr
Mim-Arg-Tyr
Pro-His-Tyr
Mim-His-Tyr
Tyr-Lys-Tyr
Mim-Lys-Tyr
Arg-His-Trp
Mim-His-Trp
Leu-Trp-Trp
Mim-Trp-Trp
Leu-Trp-Mim
Leu-His-Trp
Leu-His-Mim
BHT
Mimosine
0
20
40
60
80
100

**Fig.1**

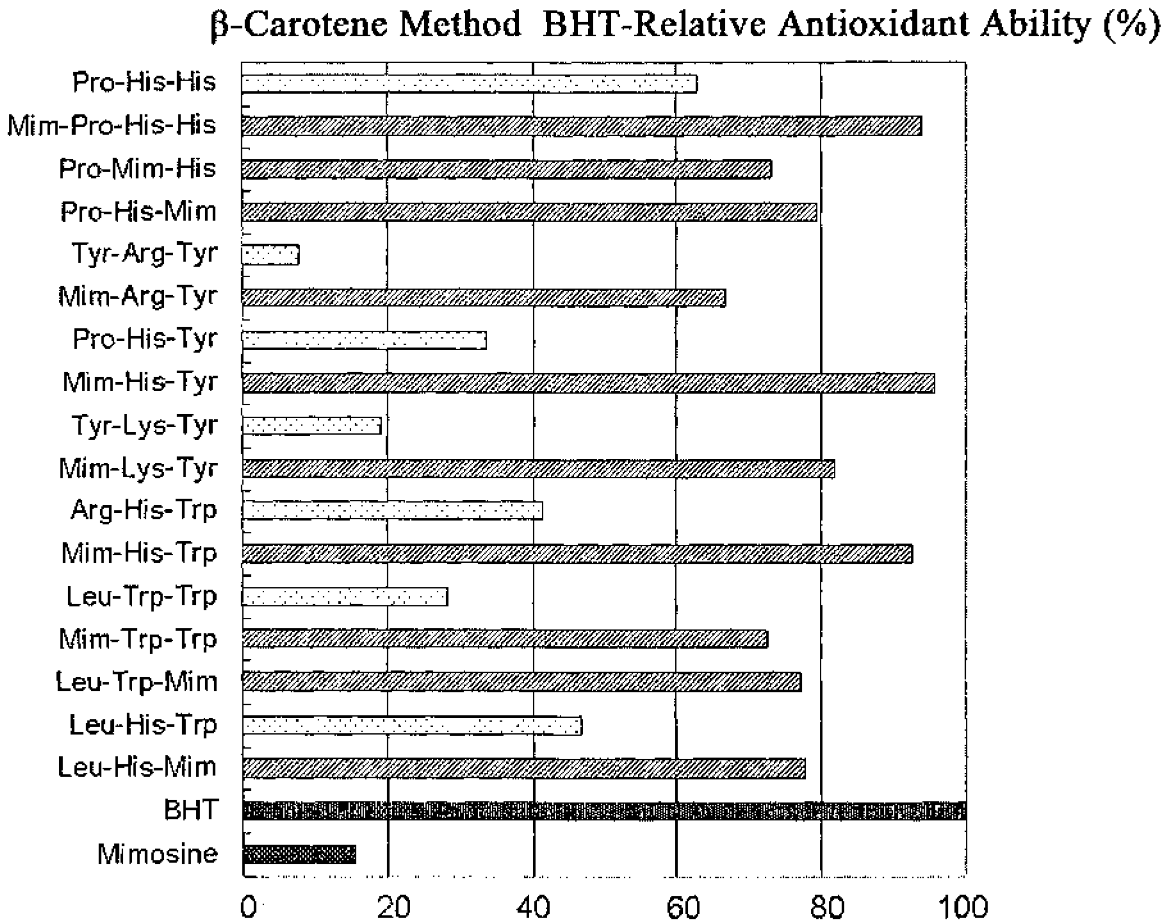
β-Carotene Method BHT-Relative Antioxidant Ability (%)
Pro-His-His
Mim-Pro-His-His
Pro-Mim-His
Pro-His-Mim
Tyr-Arg-Tyr
Mim-Arg-Tyr
Pro-His-Tyr
Mim-His-Tyr
Tyr-Lys-Tyr
Mim-Lys-Tyr
Arg-His-Trp
Mim-His-Trp
Leu-Trp-Trp
Mim-Trp-Trp
Leu-Trp-Mim
Leu-His-Trp
Leu-His-Mim
BHT
Mimosine
0
20
40
60
80
100

Fig.2

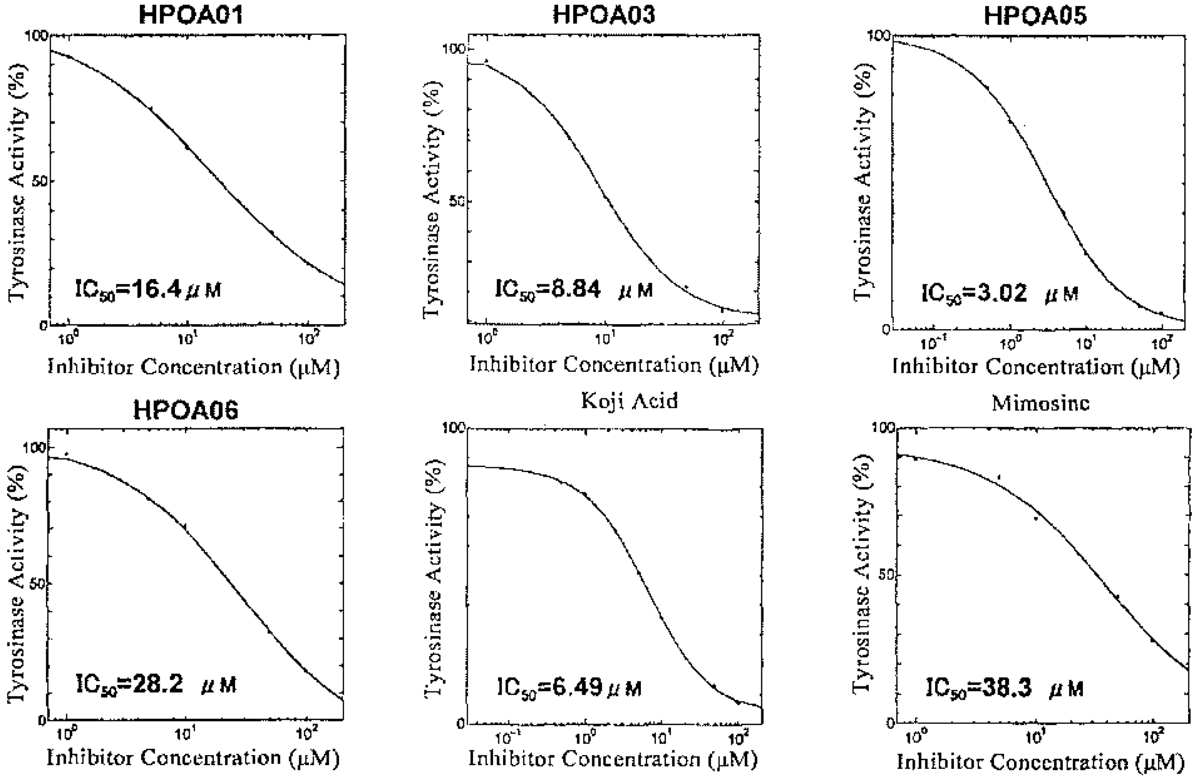

HPOA01
IC50=16.4 μM
HPOA03
IC50=8.84 μM
HPOA05
IC50=3.02 μM
HPOA06
IC50=28.2 μM
Koji Acid
IC50=6.49 μM
Mimosine
IC50=38.3 μM
Tyrosinase Activity (%)
Inhibitor Concentration (μM)


**Fig.3**

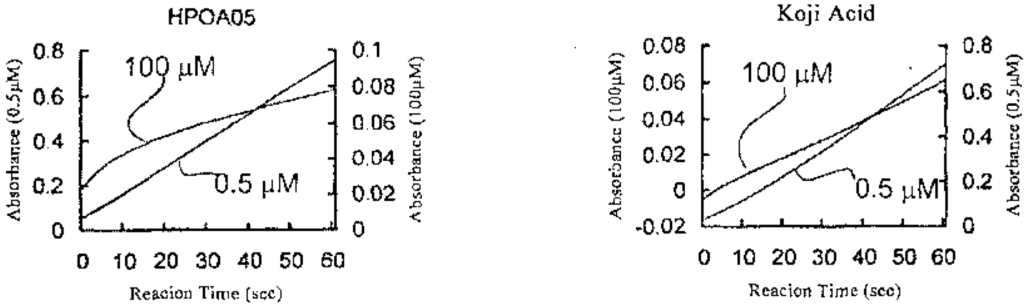

HPOA05
100 μM
0.5 μM
Absorbance (0.5μM)
Absorbance (100μM)
Reacion Time (sec)
Koji Acid
100 μM
0.5 μM
Absorbance (100μM)
Absorbance (0.5μM)
Reacion Time (sec)


**Fig.4**

**INTERNATIONAL SEARCH REPORT**

International application No.
PCT/JP2010/054112

A. CLASSIFICATION OF SUBJECT MATTER
*A61K31/4412*(2006.01)i, *A61K8/49*(2006.01)i, *A61K8/64*(2006.01)i, *A61K36/00*(2006.01)i, *A61K38/00*(2006.01)i, *A61P9/00*(2006.01)i, *A61P17/00*(2006.01)i, *A61P17/18*(2006.01)i, *A61P35/00*(2006.01)i, *A61P39/06*(2006.01)i,
According to International Patent Classification (IPC) or to both national classification and IPC

B. FIELDS SEARCHED
Minimum documentation searched (classification system followed by classification symbols)
A61K31/4412, A61K8/49, A61K8/64, A61K36/00, A61K38/00, A61P9/00, A61P17/00, A61P17/18, A61P35/00, A61P39/06, A61Q19/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
PubMed

C. DOCUMENTS CONSIDERED TO BE RELEVANT

| Category* | Citation of document, with indication, where appropriate, of the relevant passages | Relevant to claim No. |
|---|---|---|
| X<br>Y | CHOI, J.J. et al., GLIA, 2002, Vol.39, p.37-46 | 1-7,12,15<br>8 |
| Y | YAMASHOJI, S. et al., Agric. Biol. Chem., 1980, Vol.44, No.11, p.2735-2736 | 8 |
| Y | NGUYEN, S. D. et al., Archives of Biochemistry and Biophysics, 2006, Vol.451, p.34-42 | 8 |
| A | SAITO, K. et al., J. Agric. Food Chem., 2003, Vol.51, p.3668-3674 | 1-8,12,15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 May, 2010 (21.05.10) | 01 June, 2010 (01.06.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/054112

Continuation of A. CLASSIFICATION OF SUBJECT MATTER
(International Patent Classification (IPC))

*A61Q19/02*(2006.01)i

(According to International Patent Classification (IPC) or to both national classification and IPC)

Form PCT/ISA/210 (extra sheet) (July 2009)

**INTERNATIONAL SEARCH REPORT**

International application No.
PCT/JP2010/054112

**Box No. II Observations where certain claims were found unsearchable (Continuation of item 2 of first sheet)**

This international search report has not been established in respect of certain claims under Article 17(2)(a) for the following reasons:

1. [X] Claims Nos.: 16-18
because they relate to subject matter not required to be searched by this Authority, namely:
Claims 16-18 involve "methods for treatment of the human body or animal body by surgery or therapy".

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
It is considered that claims 1-15 include the following three groups of inventions.
Invention 1: Inventions relating to an antioxidant agent comprising mimosine or a method for utilizing the antioxidant agent (parts of claims 1-7, claim 8, and parts of claims 12 and 15).
Invention 2: Inventions relating to an antioxidant agent comprising a mimosine-containing peptide or a method for utilizing the antioxidant agent (parts of claims 1-7 and parts of claims 12 and 15).
Invention 3: Inventions relating to a tyrosinase inhibitor comprising a mimosine-containing peptide (Claims 9-11, 13 and 14).

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
Parts of 1-7, 8, and parts of 12 and 15

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008210592 A
- JP 63295502 A


### Non-patent literature cited in the description


- Recent Development of Food Factors For The Disease Prevention. CMC Publishers, 1998
- **Chen, H-M. ; Muramoto, K. ; Yamauchi, F. ; Nokihara, K.** *J. Agric. Food Chem.,* 1996, vol. 44, 2619-2623
- **Tawata S.** Pesticide and Alternatives. Elservier Science Publishers, 541-544
- **W.D. DeWys ; T.C.Hall.** *europ. J. Cencer,* 1973, vol. 9, 218-283
- **Saito, K. ; Jin, D-H. ; Ogawa, T. ; Muramoto, K. ; Hatakeyama, E. ; Yasuhara. T. ; Nokihara, K.** *J, Agric. Food Chemistry,* 2003, vol. 51, 3668-3674
- **Yasuhara, T. ; Nokihara, K. ; Furusho, T. ; Kataoka, E.** *Jour. Agri. Sci., Tokyo Nogyo Daigaku,* 1999, vol. 43, 260-267
- **Chen, H-M. ; Muramoto, K. ; Yamauchi, F. ; Fujimoto, K. ; Nokihara, K.** *J. Agric. Food Chem.,* 1998, vol. 46, 49-53
- **Chen, H M. ; Muramoto, K. ; Yamauchi, F. ; Nokihara, K.** *J. Agric. Food Chem.,* 1996, vol. 44, 2619-2623
- **Yasuhara, T. ; Nokihara, K.** Peptide. Kluwer Academic Publishers, 1997, 134-135
- **Nokihara, K. ; Yasuhara, T. ; Muramoto, K. ; Ando, E.** Peptide Chemistry. Protein Research Foundation, 1996, 245-248
- **Muramoto, K. ; Nokihara, K.** Recent Development of Food Factors For The Disease Prevention. CMC Publishers, 1998, 159-166
- **H-M. Chen et al.** *J. Agric. Food Chem,* 1995, vol. 43, 574-578
- **Kiyoshi NOKIHARA.** Highly Efficient Peptide Synthesis: Simultaneous and Automatic Synthesis of Multiple Peptides and Peptide Library. *Journal of Synthetic Organic Chemistry Japan,* 1994, vol. 52, 347-358
- **Kiyoshi NOKIHARA ; Yoshio YOKOMIZO.** Cleaving Off of Synthetic Peptides by Fmoc-tBu Strategy. *Shimadzu Review,* 1995, vol. 52, 1-8
- **Kiyoshi NOKIHARA.** Reagents for Highly Efficient Solid Phase Synthesis of Pcptides. *Shimadzu Review,* 1993, vol. 50, 13-24
- **Kiyoshi NOKIHARA.** Fundamental of Peptide Chemical Synthesis. *Shimadzu Review,* 1993, vol. 50, 3-12
- Development and Applications of a Novel and Practical Simultaneous Multiple Solid Phase Peptide Synthesizer. **K. Nokihara ; R. Yamamoto ; M. Hazama ; O. Wakizawa ; S. Nakamura ; M. Yamaguchi.** Peptide Chemistry. Protein Research Foundation, 1992, 203-208
- **Chan, W. C. ; White, P. D.** Fmoc Solid Phase Peptide Synthesis: A Practical Approach. Oxford University Press, 2000, 41-76

SEQUENCE LISTING

<110> HiPep Laboratories

<120> Antioxidant Agent

<130> AJW/FP6787568

<140> EP 10750900.2
<141> 2010-03-11

<150> PCT/JP2010/054112
<151> 2010-03-11

<150> JP 2009-058636
<151> 2009-03-11

<160> 14

<170> PatentIn version 3.5

<210> 1
<211> 5
<212> PRT
<213> Glycine max

<400> 1

Leu Leu Pro His His
1 5

<210> 2
<211> 4
<212> PRT
<213> Artificial

<220>
<223> synthetic peptide

<400> 2

Leu Pro His His
1

<210> 3
<211> 7
<212> PRT
<213> Gallus gallus

<400> 3

Val His His Ala Asn Glu Asn
1 5

<210> 4

<211> 8
<212> PRT
<213> Thunnus

<400> 4

Pro Ser His Asp Ala His Pro Glu
1 5

<210> 5
<211> 7
<212> PRT
<213> Thunnus

<400> 5

Val Asp His Asp His Pro Glu
1 5

<210> 6
<211> 6
<212> PRT
<213> Thunnus

<400> 6

Pro Lys Ala Val His Glu
1 5

<210> 7
<211> 4
<212> PRT
<213> Thunnus

<400> 7

Pro Ala Gly Tyr
1

<210> 8
<211> 6
<212> PRT
<213> Thunnus

<400> 8

Pro His His Ala Asp Ser
1 5

<210> 9
<211> 4
<212> PRT
<213> Thunnus

<400> 9

Val Asp Tyr Pro
1

<210> 10
<211> 11
<212> PRT
<213> Bos taurus

<400> 10

Ala Arg His Pro His Pro His Leu Ser Phe Met
1 5 10

<210> 11
<211> 16
<212> PRT
<213> Theragra chalcogramma

<220>
<221> MISC_FEATURE
<222> (3)..(3)
<223> Xaa represents 4Hyp

<220>
<221> MISC_FEATURE
<222> (6)..(6)
<223> Xaa represents 4Hyp

<220>
<221> MISC_FEATURE
<222> (9)..(9)
<223> Xaa represents 4Hyp

<220>
<221> MISC_FEATURE
<222> (12)..(12)
<223> Xaa represents 4Hyp

<220>
<221> MISC_FEATURE
<222> (15)..(15)
<223> Xaa represents 4Hyp

<400> 11

Gly Glu Xaa Gly Pro Xaa Gly Pro Xaa Gly Pro Xaa Gly Pro Xaa Gly
1 5 10 15

<210> 12
<211> 13
<212> PRT
<213> Theragra chalcogramma

<220>

```
<221>  MISC_FEATURE
<222>  (3)..(3)
<223>  Xaa represents 4Hyp

<220>
<221>  MISC_FEATURE
<222>  (6)..(6)
<223>  Xaa represents 4Hyp

<220>
<221>  MISC_FEATURE
<222>  (9)..(9)
<223>  Xaa represents 4Hyp

<220>
<221>  MISC_FEATURE
<222>  (12)..(12)
<223>  Xaa represents 4Hyp

<400>  12

Gly Pro Xaa Gly Pro Xaa Gly Pro Xaa Gly Pro Xaa Gly
1               5                   10


<210>  13
<211>  10
<212>  PRT
<213>  Gallus gallus

<400>  13

Leu Met Ser Tyr Met Trp Ser Thr Ser Met
1               5                   10


<210>  14
<211>  15
<212>  PRT
<213>  Gallus gallus

<400>  14

Leu Glu Leu His Lys Leu Arg Ser Ser His Trp Phe Ser Arg Arg
1               5                   10                  15
```